# EUROPÄISCHE PATENTANMELDUNG

(11) **EP 4 086 332 A1**
(43) Veröffentlichungstag der Anmeldung: **09.11.2022**
(21) Anmeldenummer: 22000122.6
(22) Anmeldetag: 04.05.2022
(51) Int. Cl.: C12M 1/107, C12M 1/12, E04B 1/66, E04H 7/18, F17C 3/02, F17C 3/12, C12M 1/00

(54) **AUSKLEIDUNGSFOLIE FÜR BETONIERTE RUNDBEHÄLTER**

(30) Priorität: 06.05.2021 DE 202021001669 U
(71) Anmelder: AGROTEL GmbH, 94152 Neuhaus/Inn (DE)
(72) Erfinder: Laner, Cyriak, 4761 Enzenkirchen (AT)

(57) **Zusammenfassung**

Die Auskleidungsfolie besteht im Wesentlichen aus einer ersten Folie (10), welche den Bereich der Wandauskleidung übernimmt und einer damit stoffschlüssig verbundenen zweiten Folie (20) für den Kronenbereich. Die Folie (10) für den Wandbereich besteht bevorzugt aus thermoplastischem Kunststoff und kann auch aus mehreren Schichten, auch unterschiedlicher Thermoplaste und auch Gewebeverstärkung bestehen. Vorteilhafterweise kann die Folie (10) zur besseren Haftung in Beton an der dem Beton zugewandten Seite auch mit einem Schlingengewebe oder einer anderen rauen Folie versehen werden um eine bessere Haftung zu gewährleisten.

Als Folie (20) für den Kronenbereich wird bevorzugt eine aus thermoplastischem Kunststoff, oder aus Elastomer oder einer Abmischung aus Elastomer und Thermoplast eingesetzt und weist eine geringere Steifigkeit auf. Durch die geringere Steifigkeit weist die Folie (20) eine bessere Dehnbarkeit und auch eine gewisse Elastizität auf, sodass diese nach Entfernen der Schalung über den Kronenbereich gestülpt werden kann und durch die Dehnung, da der Durchmesser nach außen hin leicht zunimmt, am Außenrand des Behälters geklemmt wird.

Die Verbindung (30) der beiden Folien (10, 20) erfolgt stoffschlüssig.

## Beschreibung

Die Erfindung betrifft eine Auskleidungsfolie für die Auskleidung der Behälterwand und Behälterkrone bei betonierten Rundbehältern.

Bevorzugt wird die Folie bereits in die Schalung eingesetzt und nach dem Entfernen der Schalung über den Kronenbereich fixiert.

Behälter die damit ausgekleidet werden, sind bevorzugt große Betontanks mit mehreren Metern Durchmesser, wie sie zur Lagerung von Substraten bei Biogasanlagen eingesetzt werden.

Eine derartige Auskleidungsfolie ist in Anspruch 1 und den folgenden abhängigen Ansprüchen beschrieben.

In verschiedenen Industrien werden große Mengen an flüssigen Stoffen in betonierten Rundbehältern gelagert. Diese Behälter werden meist individuell vor Ort hergestellt und weisen unterschiedliche Durchmesser und unterschiedliche Höhen auf. Zum Schutz des Betons und/oder zur besseren Flüssigkeitsdichtigkeit werden diese Behälter entweder bereits beim Betonieren mit einer Betonschutzfolie versehen oder zu einem späteren Zeitpunkt mit einer Gewebefolie versehen. Hier ist vor allem der obere Bereich und die Behälterkrone vor aggressiven Gasen wie Schwefelwasserstoff zu schützen. Hier ist bei bestehenden Lösungen der Schutz der Behälterkrone und die gasdichte Verbindung zur Wandauskleidung meist mangelhaft gelöst.

Oftmals wird diese Verbindungsstelle nur behelfsmäßig mit Dichtmassen verschmiert, was durch auftretende Bewegungen, bzw. der Einsatzzeit zu Rissen und damit zu Gasundichtigkeiten führt.

Ebenso ist oftmals die Befestigung der Kronenabdeckung aufwendig mit entsprechenden Spannelementen zur Behälteraußenseite realisiert. Diese Spanngurte sind aufwendig zu befestigen. Daher wird öfters auch auf Kronenabdichtungen verzichtet, was eine reduzierte Lebensdauer des Behälters bedeutet, da der Beton im oberen Bereich durch die entstehenden Gase beschädigt wird und korrodiert.

Eine undichte Verbindung führt dazu, dass insbesondere in diesem Bereich Gase aus dem Behälter in den angrenzenden Betonmauerbereich eindringen und diesen schädigen.

Daher ist es Ziel der Erfindung eine Auskleidungsfolie zu schaffen, die bereits einen fest verbundenen Wand- und Kronenauskleidungsbereich aufweist und nach dem Entfernen der Schalung einfach ohne zusätzliche Befestigungsmittel montiert werden kann.

Eine derartige Auskleidungsfolie ist nachfolgend in einer bevorzugten Ausführungsform beschrieben und ist durch Anspruch 1 und die folgenden Ansprüche charakterisiert.

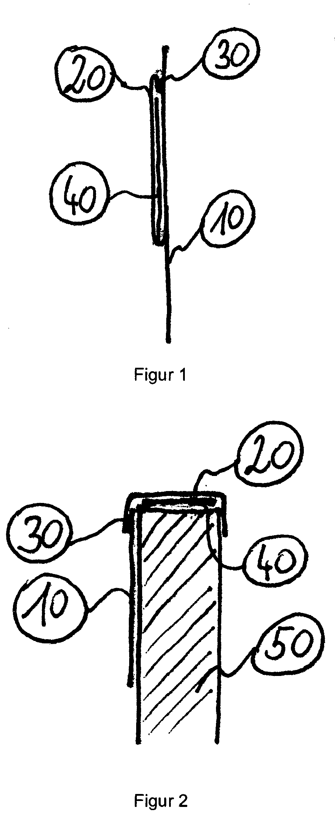

Dabei zeigt Abbildung 1 eine Schnittdarstellung einer Ausführungsvariante der Auskleidungsfolie.

In der Abbildung 2 wird die Schnittdarstellung im fertig eingebauten Zustand gezeigt.

Es wird angemerkt, dass die einzelnen Schichten bewusst voneinander beabstandet dargestellt sind, um diese zu erkennen. Im tatsächlichen Zusammenbau liegen diese Schichten ohne Zwischenraum zusammen. Dabei werden folgende Bezugszeichen verwendet:
- 10 ...: Folie zur Wandauskleidung
- 20 ...: Folie zur Kronenabdeckung
- 30 ...: stoffschlüssige Verbindung der beiden Folien
- 40 ...: Schutzschicht
- 50 ...: Betonwand

Die Auskleidungsfolie besteht im Wesentlichen aus einer ersten Folie (10), welche den Bereich der Wandauskleidung übernimmt und einer damit stoffschlüssig verbundenen zweiten Folie (20) für den Kronenbereich. Die Folie (10) für den Wandbereich besteht bevorzugt aus thermoplastischem Kunststoff, wie PP, LDPE, HDPE oder PVC und kann auch aus mehreren Schichten, auch unterschiedlicher Thermoplaste und auch Gewebeverstärkung bestehen. Vorteilhafterweise kann die Folie (10) zur besseren Haftung in Beton an der dem Beton zugewandten Seite auch mit einem Schlingengewebe oder einer anderen rauen Folie versehen werden um eine bessere Haftung zu gewährleisten.

Als Folie (20) für den Kronenbereich wird bevorzugt eine aus thermoplastischem Kunststoff, oder aus Elastomer oder einer Abmischung aus Elastomer und Thermoplast eingesetzt und weist eine geringere Steifigkeit auf. Die den Kronenbereich abdeckende Folie (20) besteht aus copolymerisiertem PP, LDPE, HDPE, PVC oder EPDM. Durch die geringere Steifigkeit weist die Folie (20) eine bessere Dehnbarkeit und auch eine gewisse Elastizität auf, sodass diese nach Entfernen der Schalung über den Kronenbereich gestülpt werden kann und durch die Dehnung, da der Durchmesser nach außen hin leicht zunimmt, am Außenrand des Behälters geklemmt wird.

Somit kann ohne zusätzliche Befestigungsgurte eine sichere Befestigung und Abdichtung auch im Kronenbereich erreicht werden.

Die Verbindung (30) der beiden Folien (10, 20) wird stoffschlüssig erreicht. Dies kann durch Verschweißen oder Verkleben erfolgen. Bevorzugt wird eine Schweißverbindung und insbesondere eine Schweißverbindung mittels Extrusionsschweißen hergestellt.

Die Oberfläche der Krone, wie auch der betonierte Rand des Behälters (50) kann leichte Unregelmäßigkeiten oder auch spitze herausragende Steine aufweisen, die die Abdeckfolie (20) beschädigen können. Dazu kann vorteilhafter Weise vor dem Überstülpen ein Vlies oder ein Schaumstoff als Schutzschicht (40) auf die Krone gelegt werden.

Für eine besonders benutzerfreundliche Variante der Auskleidungsfolie werden diese Vlies- oder Schaumstoffsegmente (40) bereits mit der Folie mitgeliefert. Hierzu kann die Abdeckfolie (20) umgefaltet werden, um in der entstehenden Tasche die Schutzsegmente (40) einzulegen und anschließend die umgeschlagene Folie (20) punktförmig stoffschlüssig zu verbinden. Durch die punktförmige Verbindung können die Schutzsegmente (40) beim Einlegen nicht unbeabsichtigt rausfallen oder beim Transport der Folie in die Schalung bereits verloren gehen.

Damit wird sichergestellt, dass nach dem Entfernen der Schalung und beim Lösen dieser Punkte die Schutzsegmente (40) zum Vorschein kommen und auch entsprechend verbaut werden und nicht vergessen werden.

Diese konfektionierte Folie kann als Rolle geliefert werden und wird dann auf der Baustelle in die Schalung auf der Innenseite eingelegt und an der Stoßfläche einmal stoffschlüssig verbunden.

Eine weitere Variante ist die aus den Planungsunterlagen definierte Länge, die sich aus dem Innendurchmesser des Behälters ergibt bereits herstellerseitig zu konfektionieren. Dazu wird die Folie auf die erforderliche Länge abgelängt und die Längsverbindung der beiden Folienenden bereits werkseitig hergestellt und auf die Baustelle geliefert.

Auf der Baustelle wird die Auskleidungsfolie in die Schalung eingelegt und anschließend der Behälter aus Beton gegossen. Nach dem Aushärten des Betons und Entfernen der Schalung wird die untere punktförmige Verbindung der Folie gelöst und die dadurch herausfallenden Schutzvliese oder Schaumstoffstreifen (40) im Bereich der Krone aufgelegt. Anschließend wird der für die Kronenabdeckung vorgesehene Folienbereich (20) angehoben und über den Rand nach außen gezogen. Da der Durchmesser nach außen hin größer wird, wird dadurch die Folie (20) großteils im elastischen Bereich gedehnt. Durch die Elastizität klemmt der Rand an der Behälteraußenwand (50) und wird dadurch ohne zusätzliche Befestigungsmittel fixiert.

Diese gesamte Montage erfolgt ohne zusätzliche Befestigungsmittel oder Werkzeuge und ist dadurch auch von ungeschultem Personal fehlerfrei durchzuführen.

Durch die stoffschlüssige Verbindung (30) zweier Folien (10, 20) wird eine gasdichte Verbindung hergestellt, sodass sowohl Behälterseitenwand, wie auch Behälterkrone zuverlässig gegenüber aggressiven Gasen geschützt werden. Durch die weniger steife, dadurch aber elastischere Folie (20) im Kronenbereich kann diese durch einfaches Überstülpen über die Krone durch die sich einstellende Spannung an der Behälteraußenwand (50) fixiert werden.

Dadurch ist ein sicherer Schutz für die Behälterinnenwand und die Behälterkrone realisiert. Die erfindungsgemäße Behälterauskleidung wird in Anspruch 1 und den abhängigen Ansprüchen 2 bis 7 beschrieben.

## Patentansprüche

1. Auskleidungsfolie zum Schutz von Behälterseitenwand und Behälterkrone bei betonierten Rundbehältern aus einer ersten Folie (10), im Bereich der Behälterwand und einer zweiten stoffschlüssig verbundenen Folie (20), die später die Krone abdeckt **dadurch gekennzeichnet, dass** die Folie (20) zur Kronenabdeckung eine geringere Steifigkeit aufweist.

2. Auskleidungsfolie nach Anspruch 1, **dadurch gekennzeichnet, dass** die Verbindung (30) der beiden Folien (10, 20) stoffschlüssig erfolgt.

3. Auskleidungsfolie nach mindestens einem der Ansprüche 1 und 2, **dadurch gekennzeichnet, dass** die Verbindung (30) der beiden Folien (10, 20) mittels Extrusionsschweißen erfolgt.

4. Auskleidungsfolie nach mindestens einem der Ansprüche 1 bis 3, **dadurch gekennzeichnet, dass** die Folie (10) der Wandauskleidung aus thermoplastischem Kunststoff aus der Gruppe PP, LDPE, HDPE oder PVC besteht.

5. Auskleidungsfolie nach mindestens einem der Ansprüche 1 bis 3, **dadurch gekennzeichnet, dass** die Folie (20) der Kronenabdeckung aus thermoplastischem oder elastomerem Kunststoff aus der Gruppe PP, LDPE, HDPE, PVC oder EPDM besteht.

6. Auskleidungsfolie nach mindestens einem der Ansprüche 1 bis 5, **dadurch gekennzeichnet, dass** die Folie (20) der Kronenabdeckung umgeschlagen und punktförmig befestigt wird.

7. Auskleidungsfolie nach mindestens einem der Ansprüche 1 bis 6, **dadurch gekennzeichnet, dass** in die durch den Umschlag entstehende Tasche ein Schutzvlies oder Schaumstoff (40) eingelegt wird.
